(19) Europäisches Patentamt
European Patent Office
Office européen des brevets

(11) Veröffentlichungsnummer : **0 072 475**
**B1**

# (12) EUROPÄISCHE PATENTSCHRIFT

(45) Veröffentlichungstag der Patentschrift : **20.08.86**

(51) Int. Cl.[4] : **C 07 C125/065**, C 07 C155/02, C 07 C149/437, A 01 N 47/12// C07C43/20, C07C149/32

(21) Anmeldenummer : **82106880.6**

(22) Anmeldetag : **30.07.82**

## (54) Carbaminsäureester, Verfahren zu deren Herstellung, Schädlingsbekämpfungsmittel, die diese Verbindungen als Wirkstoffe enthalten, sowie Verwendung solcher Verbindungen und Mittel zur Bekämpfung von Schädlingen.

(30) Priorität : **17.08.81 CH 5304/81**
**09.07.82 CH 4205/82**

(43) Veröffentlichungstag der Anmeldung : **23.02.83 Patentblatt 83/08**

(45) Bekanntmachung des Hinweises auf die Patenterteilung : **20.08.86 Patentblatt 86/34**

(84) Benannte Vertragsstaaten : **CH DE FR GB IT LI NL**

(56) Entgegenhaltungen :
**DE-A- 2 611 695**
**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE & CO. Aktiengesellschaft**
**CH-4002 Basel (CH)**

(72) Erfinder : **Zurflüh, René, Dr.**
**Dachslenbergstrasse 54**
**CH-8180 Bülach (CH)**

(74) Vertreter : **Urech, Peter, Dr. et al**
**Grenzacherstrasse 124 Postfach 3255**
**CH-4002 Basel (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 072 475 B1

**Beschreibung**

Die Erfindung betrifft Carbaminsäureester der allgemeinen Formel

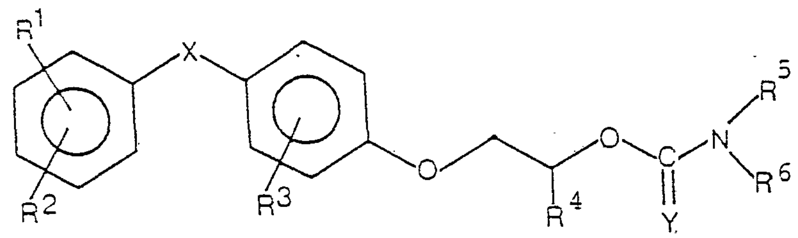

(I)

worin

$R^1$ Wasserstoff, Halogen oder Methyl,
$R^2$ Halogen, Methyl oder Trifluormethyl,
$R^3$ Wasserstoff, Halogen oder Methyl,
$R^4$ Wasserstoff oder Methyl,
$R^5$ Wasserstoff, Methyl oder Aethyl,
$R^6$ Methyl, Aethyl oder Isopropyl,
X Sauerstoff, Schwefel, Methylen oder Carbonyl

und Y Sauerstoff oder Schwefel bedeuten, wobei, falls X Schwefel, Methylen oder Carbonyl oder $R^3$ Halogen oder Methyl bedeutet, $R^2$ auch Wasserstoff sein kann.

Die Verbindungen der Formel I sind Schädlingsbekämpfungsmittel und eignen sich insbesondere zur Bekämpfung von Insekten, Acarina und Nematoden. Somit umfasst die Erfindung auch Schädlingsbekämpfungsmittel, die als Wirkstoff eine oder mehrere der Verbindungen der Formel I enthalten, Verfahren zur Herstellung dieser Verbindungen sowie die Verwendung der Verbindungen bzw. Mittel zur Schädlingsbekämpfung.

Die Deutsche Offenlegungsschrift 2.611.695 beschreibt unter anderem herbizid wirksame Mono- und Dialkyl(thio)-carbaminsäure-[2-(p-phenoxy- und p-phenylthio-phenoxy)-alkyl]ester generisch. Exemplifiziert sind solche Carbaminsäure- und Thiocarbaminsäureester, die jeweils einen Methylsubstituenten an dem die p-Phenoxy- oder p-Phenylthiophenoxygruppe tragenden Kohlenstoffatom aufweisen, wie dies beispielsweise in den Verbindungen der Beispiele 56, 57, 63, 65, 69, 73 und 75-78 der Fall ist. Von diesen exemplifizierten Verbindungen unterscheiden sich die erfindungsgemässen Verbindungen dadurch, dass sie keinen Methylsubstituenten an dem die p-Phenoxy-, p-Phenylthio-, p-Benzyl- oder p-Benzoylphenoxygruppe tragenden Kohlenstoffatom aufweisen.

Der Ausdruck « Halogen » in der obigen Definition der Verbindungen der Formel I umfasst Fluor, Chlor, Brom oder Jod. Da asymmetrische Kohlenstoffatome vorhanden sein können, liegen in solchen Fällen optische Antipoden vor. Die Formel I soll demnach all diese möglichen isomeren Formen umfassen.

Hat $R^1$ oder $R^2$ die Bedeutung Halogen, so ist dies vorzugsweise Fluor, Chlor oder Brom. Hingegen ist $R^3$ in der Bedeutung Halogen vorzugsweise Fluor oder Chlor.

Eine interessante Untergruppe von Verbindungen der Formel I besteht aus solchen Verbindungen, worin $R^2$ Halogen, Methyl oder Trifluormethyl bedeutet, und, falls X Schwefel, Methylen oder Carbonyl bedeutet, auch Wasserstoff sein kann.

Unabhängig voneinander bedeuten $R^1$ vorzugsweise Wasserstoff, m-Halogen oder m-Methyl, $R^2$ vorzugsweise Wasserstoff, p-Fluor, m-Chlor-, m-Brom, m-Methyl oder m-Trifluormethyl und $R^3$ vorzugsweise Wasserstoff oder Fluor.

$R^4$ und $R^5$ bedeuten vorzugsweise je Wasserstoff.

X ist vorzugsweise Sauerstoff.

Besonders bevorzugte Verbindungen der Formel I sind :

N-Aethylcarbaminsäure-[2-[p-(m-fluorphenoxy)phenoxy]-äthyl]ester,
N-Aethylcarbaminsäure-[2-[p-(p-fluorphenoxy)phenoxy]-äthyl]ester,
N-Aethylcarbaminsäure-[2-(o-fluor-p-phenoxy-phenoxy)-äthyl]ester und
N-Aethylcarbaminsäure-[2-[p-(m-chlorphenoxy)phenoxy]-äthyl]ester.

Weitere bevorzugte Verbindungen der Formel I sind :

N-Methylcarbaminsäure-[2-[p-(p-fluorphenoxy)phenoxy]-äthyl]ester,
N-Aethylthiocarbaminsäure-[2-[p-(m-bromphenoxy)-phenoxy]-äthyl]ester,
N-Aethylthiocarbaminsäure-[2-[p-(p-fluorphenoxy)-phenoxy]-äthyl]ester,
N-Aethylcarbaminsäure-[2-[p-(α,α,α-trifluor-m-tolyloxy)phenoxy]-äthyl]ester,
N-Aethylthiocarbaminsäure-[2-[p-(m-chlorphenoxy)-phenoxy]-äthyl]ester,
N-Aethylthiocarbaminsäure-[2-[p-(m-fluorphenoxy)-phenoxy]-äthyl]ester und
N-Aethylcarbaminsäure-[2-(m-fluor-p-phenoxy-phenoxy)-äthyl]ester.

Das erfindungsgemässe Verfahren zur Herstellung der Verbindungen der Formel I ist dadurch gekennzeichnet, dass man

a) zur Herstellung derjenigen Verbindungen der Formel I, worin $R^5$ Wasserstoff bedeutet, einen Alkohol der allgemeinen Formel

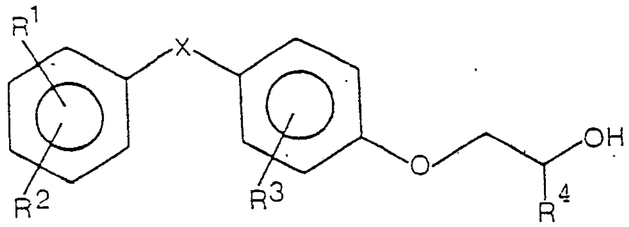

(II)

worin $R^1$, $R^2$, $R^3$, $R^4$ und X die oben angegebenen Bedeutungen besitzen, mit einem Isocyanat bzw. Isothiocyanat der allgemeinen Formel

$$R^6—N=C=Y \qquad (III)$$

worin $R^6$ und Y die oben angegebenen Bedeutungen besitzen, umsetzt,

b) zur Herstellung derjenigen Verbindungen der Formel I, worin $R^5$ Methyl oder Aethyl bedeutet, einen Alkohol der allgemeinen Formel II, wie oben definiert, mit einem Carbaminsäure- bzw. Thiocarbaminsäurehalogenid der allgemeinen Formel

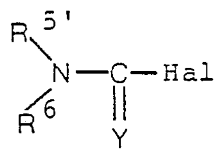

(IV)

worin $R^{5'}$ Methyl oder Aethyl und Hal Halogen, insbesondere Chlor, bedeuten und $R^6$ und Y die oben angegebenen Bedeutungen besitzen, umsetzt, oder

c) einen Alkohol der allgemeinen Formel II, wie oben definiert, mit Phosgen bzw. Thiophosgen und anschliessend einem Amin der allgemeinen Formel

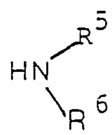

(V)

worin $R^5$ und $R^6$ die oben angegebenen Bedeutungen besitzen, umsetzt.

Die Umsetzung gemäss Verfahrensvariante a) erfolgt zweckmässigerweise in Gegenwart eines inerten Verdünnungsmittels, insbesondere eines organischen Lösungsmittels, sowie vorzugsweise in Gegenwart eines Katalysators.

Zu den bevorzugten inerten organischen Lösungsmitteln gehören Aether und ätherartige Verbindungen, wie Diäthyläther, 1,2-Dimethoxyäthan, tert.Butylmethyläther, Dioxan und Tetrahydrofuran ; Kohlenwasserstoffe, wie n-Hexan und Toluol ; halogenierte Kohlenwasserstoffe, wie Methylenchlorid, Chloroform und Tetrachlorkohlenstoff ; Ketone, wie Aceton, 2-Butanon und 3-Pentanon ; Nitrile, wie Acetonitril und Propionitril ; Formamide, wie Dimethylformamid ; und Pyridin.

Bevorzugte Katalysatoren für diese Verfahrensvariante sind tertiäre Basen, wie Triäthylamin und 1,4-Diazabicyclo-(2,2,2)octan ; und zinnorganische Verbindungen, wie Dibutyl-zinn-diacetat.

Im allgemeinen wird zwischen 0 °C und dem Siedepunkt des Reaktionsgemisches gearbeitet, vorzugsweise zwischen Raumtemperatur und dem Siedepunkt, wobei die Reaktion normalerweise innerhalb eines Tages beendet ist.

Bei der Durchführung dieser Verfahrensvariante arbeitet man vorzugsweise mit einem Ueberschuss an Isocyanat bzw. Isothiocyanat der Formel III. Zur Isolierung der so erhaltenen Verbindung der Formel I können überschüssiges Isocyanat bzw. Isothiocyanat und gegebenenfalls vorhandenes Verdünnungsmittel entfernt werden, z. B. durch Abdestillieren, und der Rückstand kann nach üblichen Methoden, z. B. durch Säulenchromatographie und/oder Kristallisieren, gereinigt werden.

Die Umsetzung gemäss Verfahrensvariante b) erfolgt zweckmässigerweise in Gegenwart eines inerten Verdünnungsmittels, insbesondere eines organischen Lösungsmittels, sowie in Gegenwart eines säurebindenden Mittels.

Als bevorzugte organische Lösungsmittel kommen die im Zusammenhang mit der Verfahrensvariante a) aufgezählten Lösungsmittel in Frage. Als säurebindende Mittel kann man alle üblichen

anorganischen und organischen säurebindenden Mittel benützen, zu denen vorzugsweise Alkalicarbonate, wie Natriumcarbonat, Kaliumcarbonat und Natriumbicarbonat ; niedere tertiäre Alkylamine, Cycloalkylamine und Arylalkylamine, insbesondere Triäthylamin ; Pyridin ; und 1,4-Diaza-bicyclo(2,2,2)octan gehören.

Die Reaktionstemperaturen können bei der Durchführung dieser Verfahrensvariante in einem grossen Bereich variiert werden. Im allgemeinen arbeitet man jedoch zwischen 0 °C und dem Siedepunkt des Reaktionsgemisches.

Man setzt vorzugsweise auf 1 Mol des Alkohols der Formel II, 1 bis 2 Mol an Carbaminsäure- bzw. Thiocarbaminsäurehalogenid der Formel IV ein. Zudem hat es sich als vorteilhaft erwiesen, das säurebindende Mittel in geringem Ueberschuss (bis ca. 30 Gewichtsprozent) einzusetzen. Die Isolierung der so erhaltenen Verbindung der Formel I kann in üblicher Weise erfolgen.

Wie im Falle der Verfahrensvariante b) erfolgt die Umsetzung gemäss Verfahrensvariante c) zweckmässigerweise in Gegenwart eines inerten Verdünnungsmittels, insbesondere eines organischen Lösungsmittels, sowie in Gegenwart eines säurebindenden Mittels.

Als bevorzugte organische Lösungsmittel kommen insbesondere Aether und ätherartige Verbindungen, wie 1,2-Dimethoxyäthan, Dioxan und Tetrahydrofuran ; Nitrile, wie Acetonitril ; Formamide, wie Dimethylformamid ; und Kohlenwasserstoffe, wie Toluol, in Frage. Man kann alle üblicherweise verwendbaren anorganischen und organischen säurebindenden Mittel zugeben, zu denen vorzugsweise die im Zusammenhang mit der Verfahrensvariante b) oben erwähnten gehören.

Die Reaktionstemperaturen können auch bei der Durchführung dieser Verfahrensvariante in einem grossen Bereich variiert werden. Im allgemeinen liegen sie zweckmässigerweise zwischen 0 °C und 100 °C, vorzugsweise zwischen 0 °C und 40 °C.

Zweckmässigerweise wird das Verfahren als ein Eintopfverfahren durchgeführt. Man setzt erst Phosgen bzw. Thiophosgen mit dem Alkohol der Formel II um, und dann gibt man zum Reaktionsgemisch das Amin der Formel V zu. Es hat sich als vorteilhaft erwiesen, auf 1 Mol des Alkohols der Formel II 1 bis 1,5 Mol Phosgen bzw. Thiophsgen und 1 bis 1,5 Mol des Amins der Formel V einzusetzen. Ferner wird das säurebindende Mittel vorzugsweise in geringem Ueberschuss, z. B. bis ca. 30 Gewichtsprozent, verwendet. Die Isolierung der so erhaltenen Verbindung der Formel I kann in üblicher Weise erfolgen.

Sofern keine gezielte Synthese zur Isolierung reiner Isomerer durchgeführt wird, fällt normalerweise ein Produkt als Gemisch zweier oder mehrerer Isomerer an. Die Isomeren können nach an sich bekannten Methoden aufgetrennt werden.

Die als Ausgangsmaterialien verwendbaren Verbindungen der Formeln II-V sind entweder bekannt oder können nach an sich bekannten Methoden hergestellt werden.

Die Alkohole der Formel II können beispielsweise durch Anlagerung eines entsprechenden 1,2-Epoxids der allgemeinen Formel

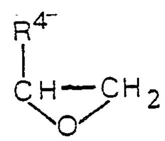

(VI)

worin $R^4$ die oben angegebene Bedeutung besitzt, an ein Phenol der allgemeinen Formel

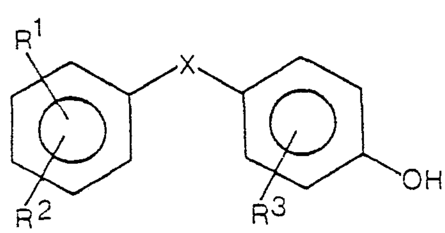

(VII)

worin $R^1$, $R^2$, $R^3$ und X die oben angegebenen Bedeutungen besitzen, hergestellt werden. Eine Ausführungsform dieser Reaktion ist beispielsweise in Houben-Weyl, Methoden der Organischen Chemie, Band VI/3, ab Seite 79 beschrieben. Zur Herstellung der Alkohole der Formel II, worin $R^4$ Wasserstoff bedeutet, kann ausserdem das Phenol der Formel VII mit Aethylencarbonat umgesetzt werden. Eine Ausführungsform dieser Reaktion findet sich in Houben-Weyl, Methoden der Organischen Chemie, Band VI/3, S. 75.

Die Isocyanate bzw. Isothiocyanate der Formel III können beispielsweise durch Umsetzung des diesbezüglichen Amins $R^6NH_2$ mit Phosgen bzw. Thiophosgen und anschliessendes Erhitzen hergestellt werden.

Zur Herstellung der Carbaminsäure- bzw. Thiocarbaminsäurechloride der Formel IV kann beispielsweise das diesbezügliche Amin $R^{5'}$ $R^6NH$ mit Phosgen bzw. Thiophosgen umgesetzt werden.

Die Verbindungen der Formel I sind ganz allgemein als Pestizide von Wert. Sie zeigen sich besonders

wertvoll zur Bekämpfung von Insekten, Acarina, insbesondere Zecken, und Nematoden, insbesondere von

Lepidoptera wie z. B. Adoxophyes spp, Tortrix viridana, Cheimatobia brumata, Lyonetia clerkella, Operophtera brumata, Lithocolletis blancardella, und anderen Miniermotten, Laspeyresia spp., Porthetria dispar, Orgyia spp., Choristoneura spp., Clysia ambiguella, Lobesia botrana, Agrotis segetum, Heliothis spp., Spodoptera spp., Ostrinia nubilalis, Ephestia spp., Galleria mellonella, Plodia interpunctella, Pectinophora gossypiella ;

Homoptera, d. h. Schild- und Schmierläusen wie z. B. Aspidiotus spp., Saissetia spp., Quadraspidiotus perniciosus, Aonidiella aurantii, Coccus spp., Unaspis spp., Lecania spp., sowie Lepidosaphes spp., Planococcus spp., Pseudococcus spp., Ceroplastes spp., Icerya purchasi, Chrysomphalus spp., Parlatoria spp., Rhizoecus spp., sowie

Zikaden wie z. B. Nephotettix spp., Laodelphax spp., Nilaparvata spp., sowie

Blattsaugern wie z. B. Psylla mali, Psylla piri, Psylla pirisuga, Psylla piricula, Trioza apicalis, sowie

Blattläusen wie z. B. Aphis fabae, Myzus persicae, sowie

Weissen Fliegen wie z. B. Trialeurodes vaporariorum, Aleurodes proletella, Bemisia tabaci ;

Diptera wie z. B. Aedes aegypti, Culex pipiens, Aedes Taeniorrhynchus, Anopheles stephensi, Calliphora spp., Musca domestica, Sciara spp., Phorbia spp., Sciaridae und Thoridae spp. ;

Coleoptera wie z. B. Oryzaephilus surinamensis, Rhizopertha dominica, Sitophilus granarius, Sitophilus oryzae, Tenebrio molitor, Tribolium castaneum, Trogoderma granarium, Lasioderma serricorne, Epilachna spp., Leptinotarsa spp., Otiorhynchus sulcatus, Diabrotica spp. und anderen Boden-Coleoptera ;

Orthoptera wie z. B. Blattella germanica, Leucophaea surinamensis, Nauphoeta cinerea, Blatta orientalis, Periplaneta americana ;

Heteroptera wie z. B. Dysdercus cingulatus, Rhodnius prolixus, Oncopeltus fasciatus, Lygus spp., Piesma spp. ;

Isoptera (Termiten-Arten) ;

Siphonaptera (Flöhe) ;

Hymenoptera wie z. B. Solenopsis invicta, Monomorium pharaonis, Atta spp. sowie echte Blattwespen, wie z. B. Athalia rosae, Hoplocampa spp., Pristiphora spp. ;

Acarina wie z. B. Tetranychus urticae, Tetranychus cinnabarinus, panonychus ulmi und anderen Tetranychiden, Eriophyiden wie Phyllocoptruta oleivora, ferner Zecken wie z. B. Amblyomma spp., Boophilus spp. und anderen Ixodoiden ;

Nematoda wie z. B. Ditylenchus dipsaci, Meliodogyne incognita, Pratylenchus penetrans, Aphelenchoides rizemabosi und Globodera rostochiensis ;

Die Verbindungen der Formel I wirken als Kontaktoder Frassgifte, oder sogar in der Dampfphase. Im Gegensatz zu den meisten bisher bekannten Schädlingsbekämpfungsmitteln, die als Toxine auf das Nervensystem der Tiere wirken und sie dadurch töten, lähmen oder vertreiben, greifen die Verbindungen der Formel I in das spezifische hormonale System des Insekten ein. Dadurch wird beispielsweise die Umwandlung zur Imago, die Ablage von entwicklungsfähigen Eiern und die Entwirklung von abgelegten normalen Eiern gestört. Bei einigen Insektenarten werden zudem die larvalen Häutungen gestört. Die Generationsfolge wird unterbrochen und die Insekten werden indirekt getötet. Die Verbindungen wirken auch auf die verschiedensten Schädlinge mit Resistenz gegenüber bisher bekannten Schädlingsbekämpfungsmitteln. Gegenüber verschiedenen nützlichen Arthropoden sind sie jedoch harmlos.

Die Verbindungen der Formel I besitzen neben pestiziden Eigenschaften auch besondere Eigenschaften, die bei der Seidenraupenzucht eine Verbesserung der Qualität und/oder Quantität des Seidenfadens erzielen. Somit lassen sie sich in der Seidenraupenzucht verwenden. Zu diesem Zwecke können die Verbindungen der Formel I beispielsweise als Zusätze zum Futter für Seidenraupen angewendet werden.

Für Wirbeltiere sind die Verbindungen der Formel I praktisch ungiftig. Die Toxizität liegt bei über

1 000 mg/kg Körpergewicht. Die Verbindungen der Formel I werden überdies leicht abgebaut. Die Gefahr einer Kumulation ist deshalb ausgeschlossen. Sie können demgemäss unbedenklich zur Bekämpfung von Schädlingen bei Tieren, Pflanzen, Vorräten und Materialien und im Wasser eingesetzt werden.

Das erfindungsgemässe Schädlingsbekämpfungsmittel ist dadurch gekennzeichnet, dass es eine wirksame Menge mindestens einer Verbindung der allgemeinen Formel I, wie oben definiert, sowie Formulierungshilfsstoffe enthält. Das Mittel enthält zweckmässigerweise zumindest einen der folgenden Formulierungshilfsstoffe :

Feste Trägerstoffe ; Lösungs- bzw. Dispersionsmittel ; Tenside (Netz- und Emulgiermittel) ; Dispergatoren (ohne Tensidwirkung) ; und Stabilisatoren.

Unter Verwendung solcher und anderer Hilfsstoffe können die Verbindungen der Formel I, also die pestiziden Wirkstoffe, in die üblichen Formulierungen übergeführt werden, wie Lösungen, Suspensionen, Emulsionen, emulgierbare konzentrate, Pasten, Schäume, Stäube, Pulver und Granulate.

Als feste Trägerstoffe kommen im wesentlichen in Frage : natürliche Mineralstoffe, wie Kaolin, Tonerden, Kieselgur, Talkum, Bentonit, Kreide, Kalkstein, Quarz, Dolomit, Attapulgit, Montmorillonit und Diatomeenerde ; synthetische Mineralstoffe, wie hochdisperse Kieselsäure, Aluminiumoxid und Silikate ; organische Stoffe, wie Cellulose, Stärke, Harnstof und Kunstharze ; und Düngemittel, wie Phosphate und Nitrate, wobei solche Trägerstoffe z. B. als Stäube, Pulver oder Granulate vorliegen können.

Als Lösungs- bzw. Dispersionsmittel kommen im wesentlichen in Frage : Aromaten, wie Toluol, Xylole, Benzol und Alkylnaphthaline ; chlorierte Aromaten und chlorierte aliphatische Kohlenwasserstoffe, wie Chlorbenzole, Chloräthylene und Methylenchlorid ; aliphatische Kohlenwasserstoffe, wie Cyclohexan und paraffine, z. B. Erdölfraktionen ; Alkohole, wie Butanol und Glykol, sowie deren Aether und Ester ; Ketone, wie Aceton, Methyläthylketon, Methylisobutylketon und Cyclohexanon ; und stark polare Lösungsmittel, wie Dimethylformamid, N-Methylpyrrolidon und Dimethylsulfoxid, wobei solche Lösungs- bzw. Dispersionsmittel vorzugsweise Flammpunkte von mindestens 30 °C und Siedepunkte von mindestens 50 °C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie halogenkohlenwasserstoffe, z. B. Dichlordifluormethan. Im Falle der Benutzung von Wasser als Lösungsmittel können z. B. auch organische Lösungsmitel als Hilfslösungsmittel verwendet werden.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid ; Fettsäureester und -äther von Zuckern oder mehrwertigen Alkoholen ; die Produkte, die aus Zuckern oder mehrwertigen Alkoholen durch Kondensation mit Aethylenoxid erhalten werden ; Blockpolymere von Aethylenoxid und Propylenoxid ; oder Alkyldimethylaminoxide.

Die Tenside können auch anionische Verbindungen darstellen, wie Seifen ; Fettsulfatester, z. B. Dodecylnatriumsulfat, Octadecylnatriumsulfat und Cetylnatriumsulfat ; Alkylsulfonate, Arylsulfonate und fettaromatische Sulfonate, wie Alkylbenzolsulfonate, z. B. Calcium-Dodecylbenzolsulfonat, und Butylnaphthalinsulfonate ; und komplexere Fettsulfonate, z. B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage : Lignin, Natrium- und Ammoniumsalze von Ligninsulfonsäure, Natriumsalze von Maleinsäureanhydrid-Diisobutylen-Copolymeren, natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z. B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel z. B. Epichlorhydrin, Phenylglycidäther und Soyaepoxide ; Antioxidantien z. B. Gallussäureester und Butylhydroxytoluol ; UV-Absorber z. B. substituierte Benzophenone, Diphenylacrylnitrilsäureester und Zimtsäureester ; und Deaktivatoren z. B. Salze der Aethylendiaminotetraessigsäure und Polyglykole.

Die erfindungsgemässen Schädlingsbekämpfungsmittel können neben den Wirkstoffen der Formel I auch andere Wirkstoffe enthalten, z. B. anderweitige Schädlingsbekämpfungsmittel, Schädlingslockstoffe, Fungizide, Bakterizide, Herbizide, Pflanzenwachstumsregulatoren und Düngemittel. Solche Kombinationsmittel eignen sich zur Verstärkung der Aktivität bzw. zur Verbreiterung des Wirkungsspektrums. Gegebenenfalls können dadurch auch Unzulänglichkeiten von bisher bekannten zugesetzten Mitteln ausgeglichen werden.

Die erfindungsgemässen Schädlingsbekämpfungsmittel enthalten im allgemeinen zwischen 0,000 5 und 95 Gewichtsprozent der Verbindung bzw. Verbindungen der Formel I als Wirkstoff, vorzugsweise zwischen 0,5 und 90 Gewichtsprozent. Sie können in einer Form vorliegen, die sich für die Lagerung und den Transport eignet. In solchen Formen, z. B. emulgierbaren Konzentraten, ist die Wirkstoffkonzentration normalerweise im höheren Bereich der obigen Konzentrationsreihe. Diese Formen können dann mit

gleichen oder verschiedenen Formulierungshilfsstoffen bis zur Wirkstoffkonzentrationen verdünnt werden, die sich für den praktischen Gebrauch eignen, und solche Konzentrationen liegen normalerweise im niedrigeren Bereich der obigen Konzentrationsreihe. Emulgierbare Konzentrate enthalten im allgemeinen 5 bis 95 Gewichtsprozent, vorzugsweise 10 bis 90 Gewichtsprozent, der Verbindung bzw. Verbindungen der Formel I. Als Anwendungsformen kommen u. a. gebrauchsfertige Lösungen, Emulsionen, Suspensionen, Schäume, Pulver, Pasten, Stäubemittel und Granulate in Frage. Die Wirkstoffkonzentrationen in solchen anwendungsfertigen Zubereitungen können in grossen Bereichen variiert werden. In Spritzbrühen können z. B. Konzentrationen zwischen 0,000 5 und 20 Gewichtsprozent vorliegen. Im Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen. Granulate, die insbesondere in der Moskito-Bekämpfung eingesetzt werden, enthalten vorzugsweise von 1 bis 10 Gewichtsprozent der Verbindung bzw. Verbindungen der Formel I als Wirkstoff.

Die erfindungsgemässen Schädlingsbekämpfungsmittel können dadurch hergestellt werden, dass man mindestens eine Verbindung der allgemeinen Formel I mit Formulierungshilfsstoffen vermischt.

Die Herstellung der Mittel kann in bekannter Weise durchgeführt werden, z. B. durch Vermischen des Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z. B. durch Zusammenmahlen ; oder man kann den festen Trägerstoff mit einer Lösung oder Suspension des Wirkstoffs imprägnieren und dann die Lösungs- oder Suspensionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z. B. als Spritzmittel eignen, übergeführt werden können.

Die Verbindungen der Formel I können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann der Verbindung der Formel I in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise enthält man emulgierbare Konzentrate bzw. gebrauchsfertige Emulsionen.

Das erfindungsgemässe Verfahren zur Bekämpfung von Schädlingen ist dadurch gekennzeichnet, dass man die zu schützenden Gegenstände oder die Schädlinge selbst mit einer erfindungsgemässen Verbindung bzw. einem erfindungsgemässen Schädlingsbekämpfungsmittel behandelt. Dieses Anwendungsverfahren kann durch Boden- oder Blattapplikation, bzw. durch Applikation auf die zu schützenden Tiere, Vorräte oder Materialien, je nach Art der zu bekämpfenden Schädlinge, durchgeführt werden. Die Bekämpfung wird beispielsweise durch Kontakt oder durch Einnahme mit der Nahrung erzielt.

Die Anwendung kann in konventioneller Weise geschehen, z. B. durch Verspritzen, Versprühen, Vernebeln, Verstäuben, Verstreuen, Eindrillen, Verräuchern, Giessen, Beizen oder Inkrustieren. Pulverförmige Präparate können z. B. als Stäubemittel mit Hilfe der üblichen Verstäubegeräte auf die Schädlinge bzw. auf die zu schützenden Gegenstände, z. B. Pflanzen oder Tiere, aufgebracht werden. Wässrige Suspensionen sind z. B. als Spritzmittel anwendbar.

Die nachfolgenden Beispiele dienen zur näheren Erläuterung der Erfindung.

I. Herstellung der Wirkstoffe der Formel I :

Beispiel 1

Zu einer Lösung von 1,9 g 2-[p-(p-Fluorphenoxy)phenoxy]-äthanol in 20 ml Toluol werden 1 Tropfen Dibutylzinn-diacetat und 0,6 g Aethylisocyanat zugegeben. Anschliessend wird das Reaktionsgemisch während 2 Stunden bei 40 °C gerührt.

Das Gemisch wird zur Aufarbeitung zuerst mit 50 ml Wasser gewaschen und die wässrige Phase mit 50 ml Diäthyläther extrahiert. Anschliessend werden die vereinigten organischen Phasen mit wasserfreiem Natriumsulfat getrocknet und eingedampft. Der zuerst ölige und dann erstarrende Rückstand wird aus Diisopropyläther umkristallisiert. Man erhält reinen N-Aethylcarbaminsäure-[2-[p-(p-fluorphenoxy)-phenoxy]-äthyl]ester ; Smp. 60-62 °C.

In Analogie zu obigem Verfahren erhält man aus :

2-[p-(p-Fluorphenoxy)phenoxy]-äthanol und Isopropylisocyanat den N-Isopropylcarbaminsäure-[2-[p-(p-fluorphenoxy)phenoxy]-äthyl]ester ; Smp. 78-79 °C.

2-[p-(p-Fluorphenoxy)phenoxy]-äthanol und Methylisocyanat den N-Methylcarbaminsäure-[2-[p-(p-fluorphenoxy)phenoxy]-äthyl]ester ; Smp. 85-87 °C.

2-[p-(α,α,α-Trifluor-m-tolyloxy)phenoxy]-äthanol und Aethylisocyanat den N-Aethylcarbaminsäure-[2-[p-(α,α,α-trifluor-m-tolyloxy)phenoxy]-äthyl]ester ; Smp. 66-68 °C.

2-[p-(m-Fluorphenoxy)phenoxy]-äthanol und Aethylisocyanat den N-Aethylcarbaminsäure-[2-[p-(m-fluorphenoxy)phenoxy]-äthyl]ester ; Smp. 58-60 °C.

1-[p-(m-Fluorphenoxy)phenoxy]-2-propanol und Methylisocyanat den N-Methylcarbaminsäure-[2-[p-(m-fluorphenoxy)phenoxy]-1-methyl-äthyl]ester ; $n_D^{20}$ 1,545 0.

1-[p-(m-Fluorphenoxy)phenoxy]-2-propanol und Aethylisocyanat den N-Aethylcarbaminsäure-[2-[p-(m-fluorphenoxy)phenoxy]-1-methyl-äthyl]ester ; $n_D^{20}$ 1,539 2.

2-(p-Benzoylphenoxy)-äthanol und Aethylisocyanat den N-Aethylcarbaminsäure-[2-(p-benzoylphenoxy)-äthyl]ester ; Smp. 87-90 °C.

2-(p-Benzylphenoxy)-äthanol und Aethylisocyanat den N-Aethylcarbaminsäure-[2-(p-benzylphenoxy)-äthyl]-ester ; Smp. 76-77 °C.

2-[p-(m-Chlorphenoxy)phenoxy]-äthanol und Aethylisocyanat den N-Aethylcarbaminsäure-[2-[p-(m-chlorphenoxy)phenoxy]-äthyl]ester ; Smp. 76-78 °C.

2-[p-(3,5-Dichlorphenoxy)phenoxy]-äthanol und Aethylisocyanat den N-Aethylcarbaminsäure-[2-[p-(3,5-dichlorphenoxy)phenoxy]-äthyl]ester ; Smp. 82-84 °C.

2-[p-(m-Tolyloxy)phenoxy]-äthanol und Aethylisocyanat den N-Aethylcarbaminsäure-[2-[p-(m-tolyloxy)phenoxy]-äthyl]ester ; Smp. 46-48 °C.

2-[p-(3-Chlor-5-fluorphenoxy)phenoxy]-äthanol und Aethylisocyanat den N-Aethylcarbaminsäure-[2-[p-(3-chlor-5-fluorphenoxy)phenoxy]-äthyl]ester ; Smp. 80-81 °C.

2-[p-(m-Chlor-p-fluorphenoxy)phenoxy]-äthanol und Aethylisocyanat den N-Aethylcarbaminsäure-[2-[p-(m-chlor-p-fluorphenoxy)phenoxy]-äthyl]ester ; Smp. 70-71,5 °C.

2-[o-Fluor-p-phenoxy-phenoxy]-äthanol und Aethylisocyanat den N-Aethylcarbaminsäure-[2-(o-fluor-p-phenoxy-phenoxy)-äthyl]ester ; $n_D^{20}$ 1,547 2.

2-[m-Fluor-p-phenoxy-phenoxy]-äthanol und Aethylisocyanat den N-Aethylcarbaminsäure-[2-(m-fluor-p-phenoxy-phenoxy)-äthyl]ester ; Smp. 56-57 °C.

2-(p-Phenylmercapto-phenoxy)-äthanol und Aethylisocyanat den N-Aethylcarbaminsäure-[2-(p-phenylmercapto-phenoxy)-äthyl]ester ; Smp. 63-64 °C.

2-[p-(p-Chlorphenylmercapto)phenoxy]-äthanol und Aethylisocyanat den N-Aethylcarbaminsäure-[2-[p-(p-chlorphenylmercapto)phenoxy]-äthyl]ester ; Smp. 92-94 °C.

2-(p-Phenoxy-m-tolyloxy)-äthanol und Aethylisocyanat den N-Aethylcarbaminsäure-[2-(p-phenoxy-m-tolyloxy)-äthyl]ester ; Smp. 52-54 °C.

2-[p-(3,5-Xylyloxy)phenoxy]-äthanol und Aethylisocyanat den N-Aethylcarbaminsäure-[2-[p-(3,5-xylyloxy)phenoxy]-äthyl]ester ; Smp. 67-68 °C.

## Beispiel 2

Zu einer Lösung von 2,3 g 2-[p-(p-Fluorphenoxy)phenoxy]-äthanol in 10 ml Pyridin werden 50 mg 1,4-Diazabicyclo(2,2,2) octan und 0,9 g Aethylisothiocyanat zugegeben. Dann lässt man während 16 Stunden bei 80 °C rühren. Das abgekühlte Gemisch wird auf 50 ml Eiswasser gegeben und dreimal mit je 80 ml Essigester extrahiert. Die Extrakte werden zweimal mit je 50 ml Wasser und mit 50 ml Kochsalzlösung gewaschen, über wasserfreiem Natriumsulfat getrocknet und eingedampft.

Durch Chromatographie an Kieselgel mit n-Hexan-Essigester (2 : 1) erhält man reinen N-Aethylthiocarbaminsäure-[2-[p-(p-fluorphenoxy)phenoxy]-äthyl]ester ; Smp. 73-75 °C.

In Analogie zu obigem Verfahren erhält man aus :

2-[p-(m-Bromphenoxy)phenoxy]-äthanol und Aethylisothiocyanat den N-Aethylthiocarbaminsäure-[2-[p-(m-bromphenoxy)phenoxy]-äthyl]ester ; Smp. 74-76 °C.

2-[p-(m-Tolyloxy)phenoxy]-äthanol und Aethylisothiocyanat den N-Aethylthiocarbaminsäure-[2-[p-(m-tolyloxy)phenoxy]-äthyl]ester ; Smp. 72-73 °C.

2-[p-(m-Chlorphenoxy)phenoxy]-äthanol und Aethylisothiocyanat den N-Aethylthiocarbaminsäure-[2-[p-(m-chlorphenoxy)phenoxy]-äthyl]ester ; Smp. 81-83 °C.

2-[p-(m-Fluorphenoxy)phenoxy]-äthanol und Aethylisothiocyanat den N-Aethylthiocarbaminsäure-[2-[p-(m-fluorphenoxy)phenoxy]-äthyl]ester ; Smp. 102-103 °C.

## Beispiel 3

Zu einer Suspension von 0,5 g Natriumhydrid (55 % in Oel) in 5 ml Dimethylformamid wird eine Lösung von 2,5 g 2-[p-(p-Fluorphenoxy)phenoxy]-äthanol in 25 ml Dimethylformamid während 20 Minuten unter Rühren zugetropft. Dann lässt man 4 Stunden bei 40 °C rühren, versetzt tropfenweise mit

1,18 g Dimethylcarbamoylchlorid und lässt 16 Stunden bei 40 °C reagieren. Das abgekühlte Reaktionsgemisch wird auf 75 ml Eiswasser gegossen und dreimal mit je 50 ml Essigester extrahiert. Die Extrakte werden dreimal mit je 50 ml Wasser gewaschen, getrocknet und eingedampft. Durch Chromatographie an Kieselgel mit n-Hexan-Essigester (85 : 15) erhält man reinen N,N-Dimethylcarbaminsäure-[2-[p-(p-fluorphenoxy)phenoxy]-äthyl]ester ; $n_D^{20}$ 1,544 4.

In Analogie zu obigem Verfahren erhält man aus 2-[p-(m-Fluorphenoxy)phenoxy]-äthanol und Dimethylcarbamoylchlorid den N,N'-Dimethylcarbaminsäure-[2-[p-(m-fluorphenoxy)-phenoxy]-äthyl]ester ; $n_D^{20}$ 1,544 2.

II. Herstellung der Ausgangsmaterialien :

Beispiel 4

Zu einer Lösung von 13,8 g p-(p-Fluorphenoxy)phenol in 35 ml Dimethylformamid werden 6,8 g Aethylencarbonat und 2,3 g Tetrabutylammoniumbromid gegeben. Nach 4 stündigem Rückfluss werden weitere 1,2 g Aethylencarbonat zugegeben, und es wird nochmals 2 Stunden am Rückfluss erwärmt. Das abgekühlte Reaktionsgemisch wird auf 150 ml Eiswasser gegossen und dreimal mit je 100 ml Essigester extrahiert. Die Extrakte werden zweimal mit je 100 ml gesättigter Kochsalzlösung gewaschen, getrocknet und eingedampft. Durch Umkristallisieren aus n-Hexan-Essigester erhält man reinen 2-[p-(p-Fluorphenoxy)phenoxy]-äthanol, Smp. 74-76 °C.

In Analogie zu obigem Verfahren erhält man aus :

p-(α,α,α-Trifluor-m-tolyloxy)phenol und Aethylencarbonat den 2-[p-(α,α,α-Trifluor-m-tolyloxy)phenoxy]-äthanol ; $n_D^{20}$ 1,531 5.

p-(m-Bromphenoxy)phenol und Aethylencarbonat den 2-[p-(m-Bromphenoxy)phenoxy]-äthanol ; $n_D^{20}$ 1,611.

p-(m-Fluorphenoxy)phenol und Aethylencarbonat den 2-[p-(m-Fluorphenoxy)phenoxy]-äthanol ; $n_D^{20}$ 1,567 7.

4-Hydroxy-benzophenon und Aethylencarbonat den 2-(p-Benzoylphenoxy)-äthanol ; Smp. 78-80 °C.

4-Benzylphenol und Aethylencarbonat den 2-(p-Benzylphenoxy)-äthanol ; Smp. 50-53 °C.

p-(m-Chlorphenoxy)phenol und Aethylencarbonat den 2-[p-(m-Chlorphenoxy)phenoxy]-äthanol ; $n_D^{20}$ 1,589 7.

p-(3,5-Dichlorphenoxy)phenol und Aethylencarbonat den 2-[p-(3,5-Dichlorphenoxy)phenoxy]-äthanol ; Smp. 56-58 °C.

p-(m-Tolyloxy)phenol und Aethylencarbonat den 2-[p-(m-Tolyloxy)phenoxy]-äthanol ; $n_D^{20}$ 1,575 3.

p-(3-Chlor-5-fluorphenoxy)phenol und Aethylencarbonat den 2-[p-(3-Chlor-5-fluorphenoxy)phenoxy]-äthanol ; $n_D^{20}$ 1,581 1.

p-(m-Chlor-p-fluorphenoxy)phenol und Aethylencarbonat den 2-[p-(m-Chlor-p-fluorphenoxy)phenoxy]-äthanol ; $n_D^{20}$ 1,569 8.

o-Fluor-p-phenoxy-phenol und Aethylencarbonat den 2-[o-Fluor-p-phenoxy-phenoxy]-äthanol ; Smp. 36-37 °C.

m-Fluor-p-phenoxy-phenol und Aethylencarbonat den 2-[m-Fluor-p-phenoxy-phenoxy]-äthanol ; $n_D^{20}$ 1,569 3.

p-(p-Chlorphenylmercapto)phenol und Aethylencarbonat den 2-[p-(p-Chlorphenylmercapto)phenoxy]-äthanol ; Smp. 68-70 °C.

p-Phenylmercapto-phenol und Aethylencarbonat den 2-(p-Phenylmercaptophenoxy)-äthanol ; $n_D^{21}$ 1,633 0.

Beispiel 5

3,1 g p-Phenoxy-m-cresol und 0,74 g Aethylenoxid werden in Gegenwart von 1 Tropfen 50 % Natronlauge im Autoklaven während 2 Stunden auf 150 °C erhitzt. Das abgekühlte Reaktionsgemisch wird mit Eiswasser versetzt und mit Essigester zweimal extrahiert. Die Extrakte werden mit halbgesättigter und gesättigter Kochsalzlösung gewaschen, über wasserfreiem Natriumsulfat getrocknet und eingedampft. Durch Chromatographie an Kieselgel mit n-Hexan-Essigester (2 : 1) erhält man reinen 2-(p-Phenoxy-m-tolyloxy)-äthanol ; $n_D^{20}$ 1,578 8.

In Analogie zu obigem Verfahren erhält man aus

p-(3,5-Xylyloxy)-phenol und Aethylenoxid den 2-[p-(3,5-Xylyloxy)phenoxy]-äthanol ; $n_D^{20}$ 1,575 6.

Beispiel 6

Ein Gemisch von 6,13 g p-(m-Fluorphenoxy)phenol, 2,79 g Propylenoxid und 0,12 g Triäthylamin wird während 18 Stunden bei 90 °C gerührt. Das abgekühlte Reaktionsgemisch wird anschliessend mit 100 ml Diäthyläther verdünnt und der Reihe nach zweimal mit je 50 ml 2N Salzsäure, dreimal mit je 50 ml 2N Natronlauge, mit 50 ml Wasser und mit 50 ml gesättigter Kochsalzlösung gewaschen. Nach Trocknen

über wasserfreiem Natriumsulfat und Eindampfen erhält man 1-[p-(m-Fluorphenoxy)phenoxy]-2-propanol von Smp. 37-39 °C.

Beispiel 7

32,3 g Hydrochinon werden in 250 ml Dimethylsulfoxid gelöst. Unter Rühren und ständigem Einleiten von Stickstoff werden nacheinander 100 ml Toluol und 30 g 86 % Kaliumhydroxid zugegeben. Nun wird die Bad-Temperatur auf 160 °C eingestellt und das Wasser mittels Wasserabscheider vollständig ausgetrieben. Danach wird die Bad-Temperatur weiter erhöht und das Toluol abdestilliert, bis die Innentemperatur 155 °C erreicht wird. Dann lässt man 40 g 3-Bromfluorbenzol zufliessen und hält das Gemisch 20 Stunden bei dieser Temperatur. Danach wird das Dimethylsulfoxid im Wasserstrahlvakuum abdestilliert. Der abgekühlte Rückstand wird auf Eiswasser gegossen, mit Salzsäure neutral gestellt und mit Essigester dreimal extrahiert. Die Extrakte werden zweimal mit Wasser und einmal mit gesättigter Kochsalzlösung gewaschen, über wasserfreiem Natriumsulfat getrocknet und eingedampft. Durch Chromatographie an Kieselgel mit n-Hexan-Essigester (9 : 1) erhält man p-(m-Bromphenoxy)phenol ; $n_D^{20}$ 1,601 8.

In Analogie zu obigem Verfahren erhält man aus :

Hydrochinon und 3-Chlorbenzotrifluorid unter Zusatz einer katalytischen Menge Kupferpulver das p-(α,α,α-Trifluor-m-tolyloxy)phenol, das im Kugelrohrofen bei 140 °C/0,05 Torr destilliert ; $n_D^{20}$ 1,537 0.

Beispiel 8

21,8 g Natriumhydrid (55 %ig in Oel) werden in 100 ml absolutem Pyridin vorgelegt und während 1 Stunde tropfenweise mit 54,1 g m-Kresol in 200 ml Pyridin versetzt. Nach vollständiger Reaktion werden 1,2 g Kupfer (I)-chlorid zugesetzt, und anschliessend werden 93,5 g 4-Bromanisol während 1 Stunde bei Rückflusstemperatur zugetropft. Nach 4 stündigem Erhitzen am Rückfluss wird das Pyridin abdestilliert, wobei am Schluss bis auf 170 °C Innentemperatur erwärmt wird. Nach 2 Stunden lässt man unter Eiskühlung 350 ml Wasser zutropfen, gefolgt von 500 ml Diäthyläther und weiteren 150 ml Wasser. Das Gemisch wird über Celite abgenutscht, und die Phasen werden getrennt. Die organische Phase wird der Reihe nach mit 200 ml 2N Salzsäure, dreimal mit je 100 ml 2N Natronlauge, mit 200 ml Wasser und mit 200 ml gesättigter Kochsalzlösung gewaschen. Nach Trocknen über wasserfreiem Natriumsulfat und Eindampfen erhält man ein braunrotes Oel, das zuerst über eine fünffache Menge Kieselgel mit n-Hexan-Diäthyläther filtriert und dann destilliert wird. Man erhält reines p-(m-Tolyloxy)anisol ; Sdp. 106-108 °C/0,04 Torr ; $n_D^{20}$ 1,573 8.

77 g p-(m-Tolyloxy)anisol werden in 360 ml Essigsäure gelöst und mit 300 ml 48 % Bromwasserstoffsäure versetzt. Dann lässt man 5 Stunden bei Rückflusstemperatur reagieren. Die abgekühlte Reaktionslösung wird auf 1,5 l Eiswasser gegossen und dreimal mit je 150 ml Methylenchlorid extrahiert. Die organische Lösung wird dreimal mit je 150 ml Wasser und mit 150 ml gesättigter Natriumbicarbonatlösung gewaschen, über wasserfreiem Natriumsulfat getrocknet und eingedampft. Durch Destillation des Rohproduktes erhält man reines p-(m-Tolyloxy)phenol ; Sdp. 88-89 °C/0,04 Torr.

In Analogie zu obigem Verfahren erhält man aus :

m-Fluorphenol und 4-Bromanisol das p-(m-Fluorphenoxy)anisol vom Sdp. 130-131 °C/0,2 Torr und daraus mit Bromwasserstoffsäure-Essigsäure das p-(m-Fluorphenoxy)phenol ; Smp. 53-55 °C.

2-Oxy-5-methoxy-toluol und Brombenzol das 5-Methoxy-2-phenoxy-toluol vom Sdp. 102-104 °C/0,035 Torr und Smp. 39-42 °C und daraus durch Methylätherspaltung das p-Phenoxy-m-cresol ; Smp. 96-99 °C.

Ebenfalls durch Methylätherspaltung erhält man aus p-(m-Chlor-p-fluorphenoxy)anisol das p-(m-Chlor-p-fluor-phenoxy)phenol ; $n_D^{20}$ 1,448 5.

Beispiel 9

6,1 g Phenol werden in 40 ml Toluol gelöst und mit einer Lösung von 3,3 g Kaliumhydroxid in 3 ml Wasser versetzt. Dann lässt man über Nacht am Wasserabscheider reagieren. Anschliessend wird das Toluol abdestilliert. Der feste Rückstand wird mit 10,2 g o-Fluor-p-bromanisol und 0,1 g Kupfer(I)chlorid versetzt und während 2 Stunden bei 180 °C Badtemperatur gerührt. Das abgekühlte Reaktionsgemisch wird mit je 100 ml Diäthyläther und Wasser versetzt. Die Aetherphase wird zweimal mit 50 ml 2N Natronlauge gewaschen, mit halbgesättigter und gesättigter Kochsalzlösung neutralgewaschen, über wasserfreiem Natriumsulfat getrocknet und eingedampft. Durch Destillation im Kugelrohrofen bei 104-106 °C/0,05 Torr erhält man o-Fluor-p-phenoxy-anisol ; $n_D^{20}$ 1,565 0.

Daraus erhält man durch Methylätherspaltung o-Fluor-p-phenoxy-phenol ; $n_D^{20}$ 1,579 2.

In Analogie zu obigem Verfahren erhält man aus :

p-Methoxyphenol und 1,3,5-Bromchlorfluorbenzol das p-(3-Chlor-5-fluorphenoxy)anisol ; $n_D^{20}$ 1,575 2, und daraus durch Methylätherspaltung das p-(3-Chlor-5-fluorphenoxy)phenol ; $n_D^{20}$ 1,582 1.

III. Formulierungsbeispiele :

Beispiel 10

Emulgierbares Konzentrat

| | g/l |
|---|---|
| Wirkstoff der Formel I | 250 |
| N-Methyl-2-pyrrolidon | 300 |
| Alkylphenol-Aethylenoxid-Addukt | 35 |
| Calciumsalz der Dodecylbenzolsulfonsäure | 15 |
| Cycloalkylepoxistearat | 25 |
| Aromatisches Lösungsmittel (Gemisch von $C_{10}$-Alkylbenzolen) | ad 1 000 ml |

Der Wirkstoff wird im N-Methyl-2-pyrrolidon gelöst, hernach die übrigen Zuschlagsstoffe zugesetzt, gelöst und mit dem aromatischen Lösungsmittel zur Marke gestellt. Das vorliegende Produkt wird zur Herstellung der gebrauchsfertigen Spritzbrühe in Wasser gegeben, wobei spontan eine für Stunden stabile Emulsion (O/W) entsteht.

Beispiel 11

Spritzpulver

| | Gew.% |
|---|---|
| Wirkstoff der Formel I | 25 |
| Kieselsäure, hydratisiert (ca. 87 % $SiO_2$) | 30 |
| Natrium-laurylsulfat | 2 |
| Natrium-lignosulfonat | 4 |
| Kaolin, hauptsächlich $Al_2[Si_2O_5](OH)_4$ | 39 |
| | 100 |

Der Wirkstoff wird mit den übrigen Formulierungskomponenten in einer geeigneten Vorrichtung homogen vermischt. Das entstandene Pulver wird nun in einem geeigneten Mahlaggregat (z. B. Stiften-, Hammer-, Kugel- oder Luftstrahlmühle) auf eine für eine optimale biologische Wirksamkeit notwendige Teilchengrösse feingemahlen und hernach nochmals gemischt. Das nun vorliegende Spritzpulver wird durch Wasser spontan benetzt und ergibt gut schwebefähige, gebrauchsfertige Spritzbrühen.

Beispiel 12

Granulat

| | Gew.% |
|---|---|
| Wirkstoff der Formel I | 5 |
| Tetranatriumsalz der Aethylendiaminotetraessigsäure ($Na_4$-AeDTA) | 1 |
| Bimssteingranulat 0,6-1,0 mm | 94 |
| | 100 |

In einem geeigneten Mischwerk wird das Bimssteingranulat vorgelegt und unter stetigem Rühren eine wässrige Lösung des $Na_4$-AeDTA aufgesprüht. Die Mischung wird bei 110 °C getrocknet und hernach der Wirkstoff, gelöst in einem geeigneten Lösungsmittel (wie z. B. Methylenchlorid), auf das trockene Gemisch aufgesprüht. Das Lösungsmittel wird durch Erwärmung verdampft. Es entsteht ein gut schüttbares Granulat, welches von Hand, mit geeigneten Granulatstreuern oder sogar vom Flugzeug aus, auf den Boden oder ins Wasser ausgebracht werden kann. Die poröse Struktur des Bimssteines bewirkt in vielen Fällen eine erwünschte verlangsamte Abgabe des Wirkstoffes über längere Zeit.

Beispiel 13

Ködergranulat

| | Gew.% |
|---|---|
| Wirkstoff der Formel I | 2 |
| Soyabohnenöl | 30 |
| Maiskolbengranulat 0,5-2,5 mm | 68 |
| | 100 |

Der Wirkstoff wird im Soyabohnenöl gelöst und diese Lösung in einer geeigneten Mischvorrichtung mit dem Maisgriess homogen vermischt. Es entsteht ein gut schüttbares Granulat, welches von Hand oder mit einem geeigneten Granulatstreuer auf den Boden ausgebracht werden kann. Es dient vornehmlich zur Bekämpfung von Feuerameisen.

**Patentansprüche**

1. Verbindungen der allgemeinen Formel

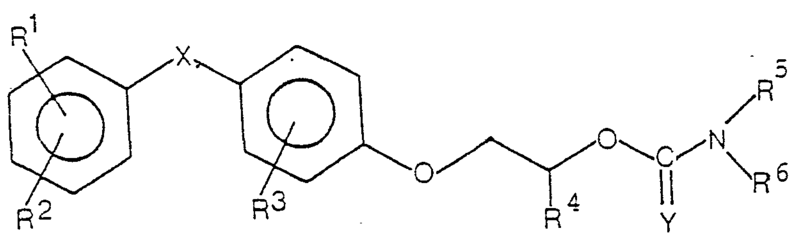

(I)

worin

$R^1$ Wasserstoff, Halogen oder Methyl,
$R^2$ Halogen, Methyl oder Trifluormethyl,
$R^3$ Wasserstoff, Halogen oder Methyl,
$R^4$ Wasserstoff oder Methyl,
$R^5$ Wasserstoff, Methyl oder Aethyl,
$R^6$ Methyl, Aethyl oder Isopropyl,
X Sauerstoff, Schwefel, Methylen oder Carbonyl
und Y Sauerstoff oder Schwefel bedeuten,
wobei, falls X Schwefel, Methylen oder Carbonyl oder $R^3$ Halogen oder Methyl bedeutet, $R^2$ auch Wasserstoff sein kann.

2. Verbindungen nach Anspruch 1, worin $R^2$ Halogen, Methyl oder Trifluormethyl bedeutet und, falls X Schwefel, Methylen oder Carbonyl bedeutet, auch Wasserstoff sein kann.

3. Verbindungen nach Anspruch 2, worin $R^1$ Wasserstoff, m-Halogen oder m-Methyl bedeutet.

4. Verbindungen nach Anspruch 2 oder 3, worin $R^2$ p-Fluor, m-Chlor, m-Brom, m-Methyl oder m-Trifluormethyl bedeutet.

5. Verbindungen nach Anspruch 1, worin $R^2$ Wasserstoff bedeutet.

6. N-Aethylcarbaminsäure-[2-[p-(m-fluorphenoxy)phenoxy]-äthyl]ester.

7. N-Aethylcarbaminsäure-[2-[p-(p-fluorphenoxy)phenoxy]-äthyl]ester.

8. N-Aethylcarbaminsäure-[2-(o-fluor-p-phenoxy-phenoxy)äthyl]ester.

9. N-Aethylcarbaminsäure-[2-[p-(m-chlorphenoxy)phenoxy]-äthyl]ester.

10. Eine Verbindung, ausgewählt aus :

N-Isopropylcarbaminsäure-[2-[p-(p-fluorphenoxy)phenoxy]-äthyl]ester,
N-Methylcarbaminsäure-[2-[p-(p-fluorphenoxy)phenoxy]-äthyl]ester,
N-Aethylcarbaminsäure-[2-[p-(α,α,α-trifluor-m-tolyloxy)phenoxy]-äthyl]ester,
N-Methylcarbaminsäure-[2-[p-(m-fluorphenoxy)phenoxy]-1-methyl-äthyl]ester,
N-Aethylcarbaminsäure-[2-[p-(m-fluorphenoxy)phenoxy]-1-methyl-äthyl]ester,
N-Aethylcarbaminsäure-[2-(p-benzoylphenoxy)-äthyl]ester,
N-Aethylcarbaminsäure-[2-(p-benzylphenoxy)-äthyl]ester,
N-Aethylcarbaminsäure-[2-[p-(3,5-dichlorphenoxy)phenoxy-äthyl]ester,
N-Aethylcarbaminsäure-[2-[p-(m-tolyloxy)phenoxy]-äthyl]ester,
N-Aethylcarbaminsäure-[2-[p-(3-chlor-5-fluorphenoxy)phenoxy]-äthyl]ester,
N-Aethylcarbaminsäure-[2-[p-(m-chlor-p-fluorphenoxy)phenoxy]-äthyl]ester,
N-Aethylcarbaminsäure-[2-(m-fluor-p-phenoxy-phenoxy)-äthyl]ester,
N-Aethylthiocarbaminsäure-[2-[p-(p-fluorphenoxy)phenoxy]-äthyl]ester,
N-Aethylthiocarbaminsäure-[2-[p-(m-bromphenoxy)phenoxy]-äthyl]ester,
N-Aethylthiocarbaminsäure-[2-[p-(m-tolyloxy)phenoxy]äthyl]ester,
N-Aethylthiocarbaminsäure-[2-[p-(m-chlorphenoxy)phenoxy]-äthyl]ester,
N-Aethylthiocarbaminsäure-[2-[p-(m-fluorphenoxy)phenoxy]-äthyl]ester und
N,N-Dimethylcarbaminsäure-[2-[p-(p-fluorphenoxy)phenoxy]-äthyl]ester.

11. Eine Verbindung, ausgewählt aus :

N-Aethylcarbaminsäure-[2-(p-phenylmercapto-phenoxy)-äthyl]ester,
N-Aethylcarbaminsäure-[2-[p-(p-chlorphenylmercapto)-phenoxy]-äthyl]ester,
N-Aethylcarbaminsäure-[2-(p-phenoxy-m-tolyloxy)-äthyl]ester und
N-Aethylcarbaminsäure-[2-[p-(3,5-xylyloxy)phenoxy]-äthyl]ester.

12. Schädlingsbekämpfungsmittel, dadurch gekennzeichnet, dass es eine wirksame Menge mindestens einer Verbindung der allgemeinen Formel I gemäss Anspruch 1 sowie Formulierungshilfsstoffe enthält.

13. Schädlingsbekämpfungsmittel nach Anspruch 12, worin $R^2$ in der Formel I Halogen, Methyl oder Trifluormethyl bedeutet und, falls X Schwefel, Methylen oder Carbonyl bedeutet, auch Wasserstoff sein kann.

14. Schädlingsbekämpfungsmittel, dadurch gekennzeichnet, dass es eine wirksame Menge N-Aethylcarbaminsäure-[2-[p-(m-fluorphenoxy)phenoxy]-äthyl]ester oder N-Aethylcarbaminsäure-[2-[p-(p-fluorphenoxy)phenoxy]-äthyl]ester oder N-Aethylcarbaminsäure-[2-(o-fluor-p-phenoxy-phenoxy)-äthyl]ester oder N-Aethylcarbaminsäure-[2-[p-(m-chlorphenoxy)phenoxy]-äthyl]ester sowie Formulierungshilfsstoffe enthält.

15. Verfahren zur Herstellung von Verbindungen der allgemeinen Formel I gemäss Anspruch 1, dadurch gekennzeichnet, dass man

a) zur Herstellung derjenigen Verbindungen der Formel I, worin $R^5$ Wasserstoff bedeutet, einen Alkohol der allgemeinen Formel

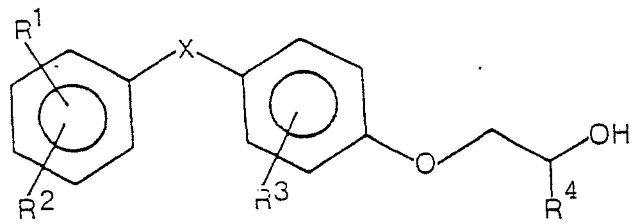

(II)

worin $R^1$, $R^2$, $R^3$, $R^4$ und X die in Anspruch 1 angegebenen Bedeutungen besitzen, mit einem Isocyanat bzw. Isothiocyanat der allgemeinen Formel

$$R^6—N=C=Y \qquad (III)$$

worin $R^6$ und Y die in Anspruch 1 angegebenen Bedeutungen besitzen, umsetzt,

b) zur Herstellung derjenigen Verbindungen der Formel I, worin $R^5$ Methyl oder Aethyl bedeutet, einen Alkohol der allgemeinen Formel II, wie oben definiert, mit einem Carbaminsäure- bzw. Thiocarbaminsäurehalogenid der allgemeinen Formel

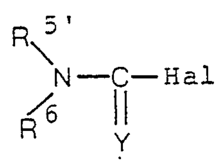

(IV)

worin $R^{5'}$ Methyl oder Aethyl und Hal Halogen bedeuten und $R^6$ und Y die in Anspruch 1 angegebenen Bedeutungen besitzen, umsetzt, oder

c) einen Alkohol der allgemeinen Formel II, wie oben definiert, mit Phosgen bzw. Thiophosgen und anschliessend einem Amin der allgemeinen Formel

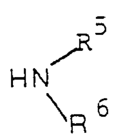

(V)

worin $R^5$ und $R^6$ die in Anspruch 1 angegebenen Bedeutungen besitzen, umsetzt.

16. Verfahren nach Anspruch 15, worin $R^2$ in den Formeln I und II Halogen, Methyl oder Trifluormethyl bedeutet und, falls X Schwefel, Methylen oder Carbonyl bedeutet, auch Wasserstoff sein kann.

17. Verfahren zur Bekämpfung von Schädlingen, dadurch gekennzeichnet, dass man die zu schützenden Gegenstände oder die Schädlinge selbst mit mindestens einer der in Anspruch 1 genannten Verbindungen bzw. mit einem in Anspruch 12 genannten Schädlingsbekämpfungsmittel behandelt.

18. Verwendung einer der in Anspruch 1 genannten Verbindungen bzw. eines in Anspruch 12 genannten Schädlingsbekämpfungsmittels zur Bekämpfung von Schädlingen.

**Claims**

1. Compounds of the general formula

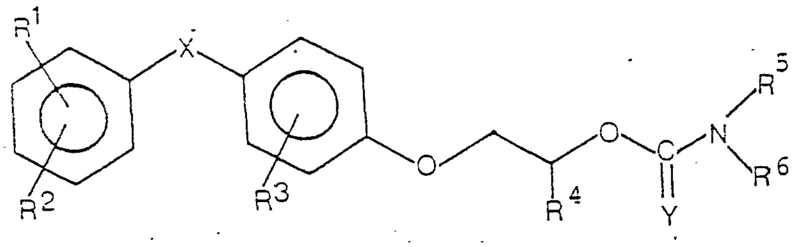

(I)

wherein
R[1] signifies hydrogen, halogen or methyl,
$R^2$ signifies halogen, methyl or trifluoromethyl,
$R^3$ signifies hydrogen, halogen or methyl,
$R^4$ signifies hydrogen or methyl,
$R^5$ signifies hydrogen, methyl or ethyl,
$R^6$ signifies methyl, ethyl or isopropyl,
X signifies oxygen, sulphur, methylene or carbonyl
and Y signifies oxygen or sulphur,
whereby, $R^2$ can also be hydrogen when X signifies sulphur, methylene or carbonyl or $R^3$ signifies halogen or methyl.

2. Compounds according to claim 1, wherein $R^2$ signifies halogen, methyl or trifluoromethyl and can also be hydrogen when X signifies sulphur, methylene or carbonyl.

3. Compounds according to claim 2, wherein $R^1$ signifies hydrogen, m-halogen or m-methyl.

4. Compounds according to claim 2 or 3, wherein $R^2$ signifies p-fluoro, m-chloro, m-bromo, m-methyl or m-trifluoromethyl.

5. Compounds according to claim 1, wherein $R^2$ signifies hydrogen.

6. 2-[p-(m-Fluorophenoxy)phenoxy]-ethyl N-ethylcarbamate.

7. 2-[p-(p-Fluorophenoxy)phenoxy]-ethyl N-ethylcarbamate.

8. 2-(o-Fluoro-p-phenoxy-phenoxy)ethyl N-ethylcarbamate.

9. 2-[p-(m-chlorophenoxy)phenoxy]-ethyl N-ethylcarbamate.

10. A compound selected from :

2-[p-(p-Fluorophenoxy)phenoxy]-ethyl N-isopropylcarbamate,
2-[p-(p-fluorophenoxy)phenoxy]-ethyl N-methylcarbamate,
2-[p-(α,α,α-trifluoro-m-tolyloxy)phenoxy]-ethyl N-ethylcarbamate,
2-[p-(m-fluorophenoxy)phenoxy]-1-methyl-ethyl N-methylcarbamate,
2-[p-(m-fluorophenoxy)phenoxy]-1-methyl-ethyl N-ethylcarbamate,
2-(p-benzoylphenoxy)-ethyl N-ethylcarbamate,
2-(p-benzylphenoxy)-ethyl N-ethylcarbamate,
2-[p-(3,5-dichlorophenoxy)phenoxy]-ethyl N-ethylcarbamate,
2-[p-(m-tolyloxy)phenoxy]-ethyl N-ethylcarbamate,
2-[p-(3-chloro-5-fluorophenoxy)phenoxy]-ethyl N-ethylcarbamate,
2-[p-(m-chloro-p-fluorophenoxy)phenoxy]-ethyl N-ethylcarbamate,
2-(m-fluoro-p-phenoxy-phenoxy)-ethyl N-ethylcarbamate,
2-[p-(p-fluorophenoxy)phenoxy]-ethyl N-ethylthiocarbamate,
2-[p-(m-bromophenoxy)phenoxy]-ethyl N-ethylthiocarbamate,
2-[p-(m-tolyloxy)phenoxy]-ethyl N-ethylthiocarbamate,
2-[p-(m-chlorophenoxy)phenoxy]-ethyl N-ethylthiocarbamate,
2-[p-(m-fluorophenoxy)phenoxy]-ethyl N-ethylthiocarbamate and
2-[p-(p-fluorophenoxy)phenoxy]-ethyl N,N-dimethylcarbamate.

11. A compound selected from :

2-(p-Phenylmercapto-phenoxy)-ethyl N-ethylcarbamate,
2-[p-(p-chlorophenylmercapto)-phenoxy]-ethyl N-ethylcarbamate,
2-(p-phenoxy-m-tolyloxy)-ethyl N-ethylcarbamate and
2-[p-(3,5-xylyloxy)phenoxy]-ethyl N-ethylcarbamate.

12. A pest control composition, characterized in that it contains an effective amount of at least one compound of general formula I in accordance with claim 1 as well as formulation adjuvants.

13. A pest control composition according to claim 12, wherein $R^2$ in formula I signifies halogen, methyl or trifluoromethyl and can also be hydrogen when X signifies sulphur, methylene or carbonyl.

14. A pest control composition, characterized in that it contains an effective amount of 2-[p-(m-fluorophenoxy)phenoxy]-ethyl N-ethylcarbamate or 2-[p-(p-fluorophenoxy)phenoxy]-ethyl N-ethylcarbamate or 2-(o-fluoro-p-phenoxy-phenoxy)-ethyl N-ethylcarbamate or 2-[p-(m-chlorophenoxy)phenoxy]-ethyl N-ethylcarbamate as well as formulation adjuvants.

15. A process for the manufacture of compounds of general formula I in accordance with claim 1, characterized by

a) for the manufacture of those compounds of formula I in which $R^5$ signifies hydrogen, reacting an alcohol of the general formula

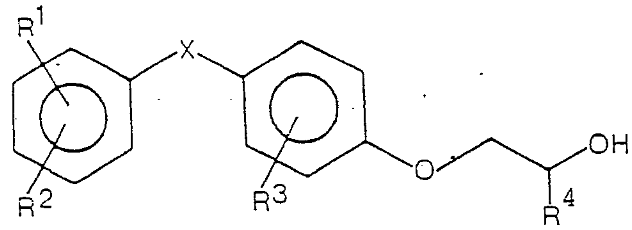

(II)

wherein $R^1$, $R^2$, $R^3$, $R^4$ and X have the significances given in claim 1, with an isocyanate or isothiocyanate of the general formula

$$R^6—N{=}C{=}Y \qquad (III)$$

wherein $R^6$ and Y have the significances given in claim 1,

b) for the manufacture of those compounds of formula I in which $R^5$ signifies methyl or ethyl, reacting an alcohol of general formula II, as defined above, with a carbamyl halide or thiocarbamyl halide of the general formula

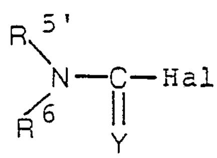

(IV)

wherein $R^{5'}$ signifies methyl or ethyl and Hal signifies halogen and $R^6$ and Y have the significances given in claim 1,

or

c) reacting an alcohol of general formula II, as defined above, with phosgene or thiophosgene and subsequently with an amine of the general formula

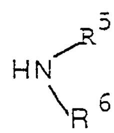

(V)

wherein $R^5$ and $R^6$ have the significances given in claim 1.

16. A process according to claim 15, wherein $R^2$ in formulae I and II signifies halogen, methyl or trifluoromethyl and can also be hydrogen when X signifies sulphur, methylene or carbonyl.

17. A method for the control of pests, characterized by treating the locus to be protected or the pests themselves with at least one of the compounds set forth in claim 1 or with a pest control composition set forth in claim 12.

18. The use of one of the compounds set forth in claim 1 or of a pest control composition set forth in claim 12 for the control of pests.

**Revendications**

1. Composés de formule générale

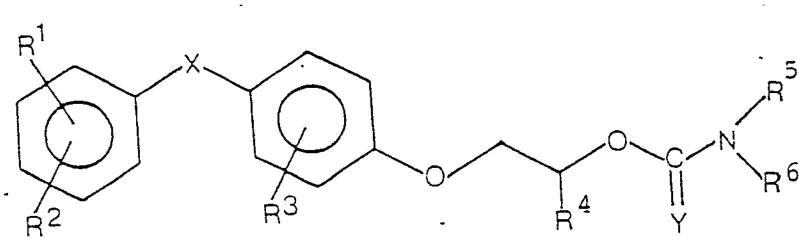

(I)

dans laquelle

$R^1$ représente l'hydrogène, un halogène ou un groupe méthyle,

$R^2$ représente un halogène, un groupe méthyle ou trifluorométhyle,

$R^3$ représente l'hydrogène, un halogène ou un groupe méthyle,

$R^4$ représente l'hydrogène ou un groupe méthyle,

$R^5$ représente l'hydrogène, un groupe méthyle ou éthyle,

$R^6$ représente un groupe méthyle, éthyle ou isopropyle,

X représente l'oxygène, le soufre, un groupe méthylène ou carbonyle,

et Y représente l'oxygène ou le soufre,

$R^2$ pouvant également représenter l'hydrogène lorsque X représente le soufre, un groupe méthylène ou carbonyle ou que $R^3$ représente un halogène ou un groupe méthyle.

2. Composés selon la revendication 1, dans lesquels $R^2$ représente un halogène, un groupe méthyle ou trifluorométhyle, et peut également représenter l'hydrogène lorsque X représente le soufre, un groupe méthylène ou carbonyle.

3. Composés selon la revendication 2, dans lesquels $R^1$ représente l'hydrogène, un halogène en position méta ou un groupe méthyle en position méta.

4. Composés selon la revendication 2 ou 3, dans lesquels $R^2$ représente le fluor en position para, le chlore en position méta, le brome en position méta, un groupe méthyle en position méta ou trifluorométhyle en position méta.

5. Composés selon la revendication 1, dans lesquels $R^2$ représente l'hydrogène.

6. Le N-éthylcarbamate de 2-[p-(m-fluorophénoxy)-phénoxy]-éthyle.

7. Le N-éthylcarbamate de 2-[p-(p-fluorophénoxy)-phénoxy]-éthyle.

8. Le N-éthylcarbamate de 2-(o-fluoro-p-phénoxyphénoxy)-éthyle.

9. Le N-éthylcarbamate de 2-[p-(m-chlorophénoxy)-phénoxy]-éthyle.

10. Un composé choisi parmi les suivants :

N-isopropylcarbamate de 2-[p-(p-fluorophénoxy)-phénoxy]-éthyle,
N-méthylcarbamate de 2-[p-(p-fluorophénoxy)-phénoxy]-éthyle,
N-éthylcarbamate de 2-[p-(α,α,α-trifluoro-m-tolyloxy)-phénoxy]-éthyle,
N-méthylcarbamate de 2-[p-(m-fluorophénoxy)-phénoxy]-1-méthyl-éthyle,
N-éthylcarbamate de 2-[p-(m-fluorophénoxy)-phénoxy]-1-méthyl-éthyle,
N-éthylcarbamate de 2-(p-benzoylphénoxy)-éthyle,
N-éthylcarbamate de 2-(p-benzylphénoxy)-éthyle,
N-éthylcarbamate de 2-[p-(3,5-dichlorophénoxy)-phénoxy]-éthyle,
N-éthylcarbamate de 2-[p-(m-tolyloxy)-phénoxy]-éthyle,
N-éthylcarbamate de 2-[p-(3-chloro-5-fluorophénoxy)-phénoxy]-éthyle,
N-éthylcarbamate de 2-[p-(m-chloro-p-fluorophénoxy)-phénoxy]-éthyle,
N-éthylcarbamate de 2-(m-fluoro-p-phénoxy-phénoxy)-éthyle,
N-éthylcarbamate de 2-[p-(p-fluorophénoxy)-phénoxy]-éthyle,
N-éthylthiocarbamate de 2-[p-(m-bromophénoxy)-phénoxy]-éthyle,
N-éthylthiocarbamate de 2-[p-(m-tolyloxy)-phénoxy]-éthyle,
N-éthylthiocarbamate de 2-[p-(m-chlorophénoxy)-phénoxy]-éthyle,
N-éthylthiocarbamate de 2-[p-(m-fluorophénoxy)-phénoxy]-éthyle, et
N,N-diméthylcarbamate de 2-[p-(p-fluorophénoxy)-phénoxy]-éthyle.

11. Un composé choisi parmi les suivants :

N-éthylcarbamate de 2-(p-phénylmercapto-phénoxy)-éthyle,
N-éthylcarbamate de 2-[p-(p-chlorophénylmercapto)-phénoxy]-éthyle,
N-éthylcarbamate de 2-(p-phénoxy-m-tolyloxy)-éthyle, et
N-éthylcarbamate de 2-[p-(3,5-xylyloxy)-phénoxy]-éthyle.

12. Produit pesticide caractérisé en ce qu'il contient une quantité efficace d'au moins un composé de formule générale I selon la revendication 1, et des produits auxiliaires de formulation.

13. Produit pesticide selon la revendication 12, dans lequel le symbole $R^2$ de la formule I représente un halogène, un groupe méthyle ou trifluorométhyle, et peut également représenter l'hydrogène lorsque X représente le soufre, un groupe méthylène ou carbonyle.

14. Produit pesticide caractérisé en ce qu'il contient une quantité efficace de N-éthylcarbamate de 2-[p-(m-fluorophénoxy)-phénoxy]-éthyle ou de N-éthylcarbamate de 2-[p-(p-fluorophénoxy)-phénoxy]-éthyle ou de N-éthylcarbamate de 2-(o-fluoro-p-phénoxy-phénoxy)-éthyle ou de N-éthylcarbamate de 2-[p-(m-chlorophénoxy)-phénoxy]-éthyle et des produits auxiliaires de formulation.

15. Procédé de préparation des composés de formule générale I selon la revendication 1, caractérisé en ce que :

a) pour préparer des composés de formule I dans laquelle $R^5$ représente l'hydrogène, on fait réagir un alcool de formule générale

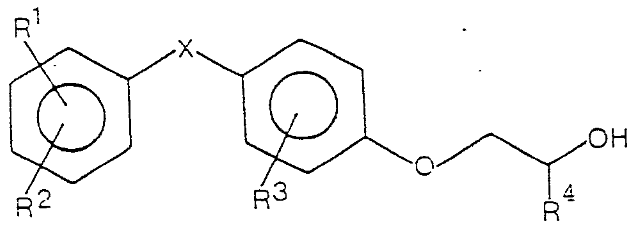

(II)

dans laquelle $R^1$, $R^2$, $R^3$, $R^4$ et X ont les significations indiquées dans la revendication 1, avec un isocyanate ou isothiocyanate respectivement de formule générale

$$R^6\text{—}N{=}C{=}Y \qquad \text{(III)}$$

dans laquelle $R^6$ et Y ont les significations indiquées dans la revendication 1,

b) pour préparer les composés de formule I dans laquelle $R^5$ représente un groupe méthyle ou éthyle, on fait réagir un alcool de formule générale II définie ci-dessus avec un halogénure d'acide carbamique ou thiocarbamique respectivement, de formule générale

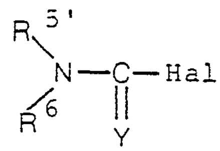

(IV)

dans laquelle $R^{5'}$ représente un groupe méthyle ou éthyle, et Hal représente un halogène, et $R^6$ et Y ont les significations indiquées dans la revendication 1, ou bien

c) on fait réagir un alcool de formule générale II telle que définie ci-dessus avec le phosgène ou le thiophosgène respectivement puis avec une amine de formule générale

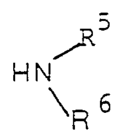

(V)

dans laquelle $R^5$ et $R^6$ ont les significations indiquées dans la revendication 1.

16. Procédé selon la revendication 15, dans lequel $R^2$ des formules I et II représente un halogène, un groupe méthyle ou trifluorométhyle et peut également représenter l'hydrogène lorsque X représente le soufre, un groupe méthylène ou carbonyle.

17. Procédé pour combattre les parasites, caractérisé en ce que l'on traite les objets à protéger ou les parasites eux-mêmes par au moins un des composés mentionnés dans la revendication 1 ou par un produit pesticide mentionné dans la revendication 12.

18. Utilisation de l'un des composés mentionnés dans la revendication 1 ou d'un produit pesticide mentionné dans la revendication 12 dans la lutte contre les parasites.